(19) Europäisches Patentamt

European Patent Office

Office européen des brevets

(11) **EP 0 657 420 B1**

(12) **EUROPÄISCHE PATENTSCHRIFT**

(45) Veröffentlichungstag und Bekanntmachung des
Hinweises auf die Patenterteilung:
**03.09.1997 Patentblatt 1997/36**

(51) Int. Cl.⁶: **C07C 269/04**

(21) Anmeldenummer: **94118822.9**

(22) Anmeldetag: **30.11.1994**

(54) **Verfahren zur Herstellung von Diurethanen und ihrer Verwendung zur Herstellung von Diisocyanaten**

Process for the preparation of diurethanes and their use in the preparation of diisocyanates

Procédé pour la préparation de diuréthanes et leur utilisation pour la préparation d'isocyanates

(84) Benannte Vertragsstaaten:
**DE ES FR GB IT NL**

(30) Priorität: **13.12.1993 DE 4342426**

(43) Veröffentlichungstag der Anmeldung:
**14.06.1995 Patentblatt 1995/24**

(73) Patentinhaber: **BAYER AG
51368 Leverkusen (DE)**

(72) Erfinder:
- **Wilmes, Oswald, Dr.
  D-51061 Köln (DE)**
- **König, Eberhard, Dr.
  D-51375 Leverkusen (DE)**
- **Nachtkamp, Klaus, Dr.
  D-40593 Düsseldorf (DE)**
- **Kysela, Ernst, Dr.
  D-51427 Bergisch-Gladbach (DE)**

(56) Entgegenhaltungen:
EP-A- 0 018 586          EP-A- 0 019 109

## Beschreibung

Die vorliegende Erfindung betrifft ein Verfahren zur Herstellung von hochreinen (cyclo)aliphatischen Diurethanen aus (cyclo)aliphatischen Diaminen, Harnstoff und Alkoholen unter Freisetzung von Ammoniak sowie die Verwendung der erhaltenen Diurethane zur Herstellung von (cyclo)aliphatischen Diisocyanaten durch thermische Urethanspaltung.

Die Urethanisierung von (cyclo)aliphatischen Aminen mit Harnstoff bzw. Carbamidsäureestern und Alkoholen ist im Prinzip bekannt. Gemäß EP-A-00 18 586 werden (cyclo)aliphatische Diurethane auf Basis niederer (cyclo)aliphatischer Alkohole (Siedepunkt des Alkohols <180°C) zwar in Ausbeuten bis über 90 % erhalten, über deren Reinheit wird aber nichts mitgeteilt. Bei Verwendung hochsiedender Alkohole (Siedepunkt des Alkohols >180°C) ist aus den Beispielen ersichtlich, daß zum Teil noch beträchtliche Anteile von Nebenprodukten (bis zu 25 %) vorhanden sind.

Nach eigenen Erfahrungen entstehen bei der in EP-A-00 18 586 beschriebenen Vorgehensweise bei Verwendung niedrig siedender Alkohole auch Oligo- und Polyharnstoffe, die bei der thermischen Urethanspaltung empfindlich stören können. Es ist anzunehmen, daß diese durch einmalige Umkristallisation nicht vollständig abgetrennt werden und das Produkt verunreinigen. Das hat zur Folge, daß die in den Beispielen der EP-A-00 18 586 erhaltenen Ausbeuten niedriger sind als die angegebenen Werte. Eigene Umkristallisationsversuche haben zudem ergeben, daß die als Verunreinigungen enthaltenen Oligo- und Polyharnstoffe bei dieser in den Beispielen der EP-A-00 18 586 angegebenen. Reinigungsoperation sogar noch angereichert werden.

In den Ausführungsbeispielen der EP-A-00 18 588, die die Verwendung von N-unsubstituierten Carbamidsäureestern an Stelle von Harnstoff als "Carbonylquelle" zum Gegenstand hat, werden im Falle der Verwendung von niedrig siedenden Alkoholen (Siedepunkt des Alkohols <180°C) keine Reinheiten mitgeteilt, die dabei erzielten Ausbeuten liegen unterhalb denen gemäß EP-00 18 586, so daß hier sogar ein erhöhter Nebenproduktgehalt angenommen werden kann.

Sowohl in EP-A-00 18 586 als auch in EP-A-00 18 588 wird zur Erhöhung der Reaktionsgeschwindigkeit die Verwendung von Katalysatoren empfohlen. Nachteilig ist, daß dabei gewisse Anteile des Katalysators im Produkt verbleiben können. Zur Durchführung der Verfahren beider Vorveröffentlichungen wird darauf hingewiesen, daß es, insbesondere bei der Umsetzung von niedermolekularen Alkoholen unter Druck, zweckmäßig sein kann, den Ammoniak mit Hilfe eines unter den Reaktionsbedingungen inerten Strippmittels, beispielsweise eines Gases wie Stickstoff, abzutrennen. Dieses Vorgehen hat aber den Nachteil, daß im Fall eines technischen Verfahrens eine zusätzliche Abgasreinigung, die einen besonderen Aufwand darstellt, erforderlich ist.

Weitere, dem nachstehend naher beschriebenen erfindungsgemäßen Verfahren, ferner liegende Verfahren zur Herstellung von Diurethanen auf Basis der entsprechenden Diaminen, Alkoholen und niedermolekularen "Carbonylquellen" werden beispielsweise in EP-A-00 27 940, EP-A-00 27 952, EP A-00 27 953, EP-A-01 26 299, EP-A-01 26 300, EP-A 03 55 443, EP-A- 05 66 925 oder DE-OS 4 231 417 beschrieben.

In den Ausführungsbeispielen dieser Vorveröffentlichungen verden im allgemeinen keine Angaben bezüglich der Reinheit der erhaltenen Diurethane gemacht. Wie eigene Untersuchungen jedoch ergaben, sind die gemäß diesen Verfahren des Standes der Technik erhaltenen Diurethane nicht ohne Zumindest teilweise vorherige Abtrennung der als Nebenprodukte mitentstandenen Oligo- und Polyharnstoffe spaltbar, es sei denn, es werden beträchtliche Ausbeuteverluste und nachteilhafte Begleiterscheinungen, wie beispielsweise harzartige Ablagerungen im Spaltreaktor in Kauf genommen.

Aufgabe der vorliegenden Erfindung war es daher, ein Verfahren zur Herstellung von hochreinen (cyclo)aliphatischen Diurethanen zur Verfügung zu stellen, die für die Herstellung von (cyclo)aliphatischen Diisocyanaten durch thermische Urethanspaltung besonders gut geeignet sind. Gegebenenfalls in geringer Menge anfallende Nebenprodukte sollten leicht abtrennbar und recyclisierbar sein.

Überraschenderweise gelingt die Lösung dieser Aufgabe mit dem nachstehend näher beschriebenen erfindungsgemäßen Verfahren, bei welchen zunächst Harnstoff mit Alkohol unter Entnahme von Ammoniak umgesetzt und dann ohne Zwischenisolierung das Diamin, gegebenenfalls in gelöster Form und unter kontinuierlicher Freisetzung von Ammoniak zur Reaktion gebracht wird. Zur Reindarstellung der Verfahrensprodukte ist lediglich die destillative Entfernung von überschüssigem Alkohol, sowie der bei erhöhter Temperatur im Vakuum flüchtigen Harnstoffderivate erforderlich.

Gegenstand der Erfindung ist ein Verfahren zur Herstellung von Diurethanen der Formel (I)

$$R\text{-}O\text{-}CO\text{-}NH\text{-}R'\text{-}NH\text{-}CO\text{-}O\text{-}R \qquad (I)$$

unter Verwendung von Diaminen der Formel (II)

$$H_2N\text{-}R'\text{-}NH_2 \qquad (II)$$

Alkoholen der Formel (III)

$$R\text{-}OH \qquad (III)$$

und Harnstoff als Ausgangsmaterialien, wobei

R     für den Rest steht, wie er durch Entfernung der Hydroxylgruppe aus einem einwertigen Alkohol mit einem Siedepunkt unter Normaldruck von unter 180°C erhalten wird und

R'     für einen zweiwertigen (cyclo)aliphatischen Kohlenwasserstoffrest mit 2 bis 18, vorzugsweise 4 bis 15 Kohlenstoffatomen steht, mit der Maßgabe, daß zwischen den beiden Stickstoffatomen mindestens 2 Kohlenstoffatome angeordnet sind,

dadurch gekennzeichnet, daß man

in einer ersten Stufe Harnstoff mit Alkohol der Formel (III) unter Einhaltung eines Molverhältnisses von Alkohol zu Harnstoff von mindestens 1:1, bei 150 bis 300°C unter Abspaltung und Entfernung aus dem Reaktionsgemisch von mindestens 10 Mol-% Ammoniak, bezogen auf die molare Menge des eingesetzten Harnstoffs, umsetzt, anschließend

in einer zweiten Stufe in diese Reaktionsmischung das Diamin der Formel (II), gegebenenfalls in in Alkohol der Formel (III) gelöster Form ein dosiert und unter kontinuierlicher Freisetzung von Ammoniak bei 150 bis 300°C umsetzt und

schließlich in einer dritten Stufe das Reaktionsgemisch destillativ von flüchtigen Bestandteilen befreit.

Gegenstand der Erfindung ist auch die Verwendung der nach diesem Verfahren erhältlichen Diurethane als Ausgangsmaterial zur Herstellung der entsprechenden Diisocyanate durch thermische Urethanspaltung.

Ausgangsmaterialien für das erfindungsgemäße Verfahren sind Diamine der bereits obengenannten Formel (II), Harnstoff und Alkohole der bereits obengenannten Formel (III).

Geeignete Diamine der Formel (II) sind beispielsweise Butandiamin-1,4, 1-Methylpentandiamin-1,5, 2-Methylpentandiamin-1,5, Hexandiamin-1,6, 2,2,4- bzw. 2,4,4-Trimethylhexandiamin-1,6, Cyclohexandiamin-1,3, Cyclohexandiamin-1,4, 2-Methyl- und/oder 4-Methylcyclo-hexandiamin-1,3 (hydriertes Toluylendiamin), 1,3- und 1,4-Diaminomethylcyclohexan, Bis-(4-aminocyclohexyl)-methan, Bis-(4-amino-3-methylcyclohexyl)-methan, 3-Aminomethyl-3,5,5-trimethyl-cyclohexylamin (Isophorondiamin) und 3- und/oder 4-Aminomethyl-1-methylcylohexylamin.

Besonders bevorzugt werden als Diamine der Formel (II) Hexandiamin-1,6 (HDA), 3-Aminomethyl-3,5,5-trimethyl-cyclohexylamin (IPDA) oder Bis-(4-aminocyclohexyl)-methan eingesetzt.

Als Alkohole der Formel (III) eignen sich beliebige aliphatische oder cycloaliphatische Alkohole, die unter Normaldruck einen unterhalb 180°C liegenden Siedepunkt aufweisen. Beispielhaft genannt seien $C_1$-$C_6$-Alkanole wie z.B. Methanol, Ethanol, n-Propanol, n-Butanol oder n-Hexanol oder Cyclohexanol. Besonders bevorzugt wird n-Butanol als Alkohol verwendet.

Zur Durchführung des erfindungsgemäßen Verfahrens wird zunächst der als Carbonylquelle dienende Harnstoff mit dem Alkohol der Formel (III) unter Abspaltung von Ammoniak zur Reaktion gebracht. Diese Umsetzung erfolgt bei 150 bis 300°C, vorzugsweise 160 bis 250°C unter Verwendung einer mindestens äquimolaren, vorzugsweise 1,2- bis 25-fach, insbesondere 1,2- bis 10-fach molaren Menge des Alkohols, bezogen auf Harnstoff, bis mindestens 10 Mol-%, vorzugsweise 30 bis 90 Mol-% Ammoniak, bezogen auf die molare Menge des eingesetzten Harnstoffs aus dem Reaktionsgemisch entwichen sind. Hieraus kann abgeleitet werden, daß mindestens 10, vorzugsweise 30 bis 90 % des bei vollständiger Umsetzung des eingesetzten Harnstoffs zum N-unsubstituierten Carbamat gemäß der Gleichung

$$H_2N\text{-}CO\text{-}NH_2 + R\text{-}OH \rightarrow H_2N\text{-}CO\text{-}O\text{-}R + NH_3$$

theoretisch zu erwartenden Ammoniaks abgespalten sind. Hierzu kann man sich beispielsweise eines mit Rührer, Rückflußkühler und Druckhalteventil ausgerüsteten Druckreaktors bedienen, wobei das Druckhalteventil so eingestellt wird, daß der im Überschuß zur Anwendung gelangende Alkohol am Rückfluß siedet und Ammoniak gasförmig entweichen kann.

In der zweiten Stufe des erfindungsgemäßen Verfahrens erfolgt die Zugabe des Diamins der Formel (II), entweder in reiner Form, gegebenenfalls als Schmelze, oder vorzugsweise in Form einer mindestens 5 gew.-%igen Lösung in einer weiteren Menge Alkohol der Formel (III). Die Zugabe des Diamins zum Reaktionsgemisch erfolgt allmählich innerhalb des Temperaturbereichs von 150 bis 300°C, vorzugsweise 160 bis 250°C, erneut unter fortgesetzte Entnahme von Ammoniak, wobei vorzugsweise ebenfalls der Druck im Reaktor so eingestellt wird, daß der im Überschuß vorliegende Alkohol am Rückfluß siedet.

Die Menge des Diamins wird hierbei im allgemeinen so bemessen, daß auf jedes Mol an zu Beginn eingesetzten Harnstoffs 0,1 bis 0,5 Mol, vorzugsweise 0,3 bis 0,5 Mol Diamin zum Einsatz gelangen. Die Gesamtmenge des in der ersten und zweiten Stufe zum Einsatz gelangenden Alkohols liegt bei 1 bis 25 Mol, vorzugsweise bei 1,2 bis 10 Mol pro Mol an zu Beginn eingesetztem Harnstoff.

Im allgemeinen wird unter den genannten Reaktionsbedingungen nachgerührt, bis die Ammoniakentwicklung nach ca. 1-2 Stunden nachläßt, anschließend wird im allgemeinen noch eine gewisse Zeit, z.B. mindestens 1 Stunde unter den genannten Reaktionsbedingungen weitergerührt, um das Reaktionsgemisch anschließend in der dritten Stufe der destillativen Aufarbeitung zuzuführen.

Bei dieser destillativen Aufarbeitung erfolgt zunächst eine destillative Entfernung des Leichtsieders (überschüssiger Alkohol) und anschließend, beispielsweise unter einem Vakuum von 0,001 bis 500 mbar, eine destillative Entfernung der in Mengen von maximal 20 Gew.-%, bezogen auf gebildetes Diurethan, vorliegenden Mittelsieder. Hierunter sind (i) der dem Alkohol entsprechende N-unsubstituierte Carbamidsäureester, (ii) N,O-disubstituierte Carbamidsäureester, deren Substituenten dem eingesetzten Alkohol entsprechen und (iii) die Di(cyclo)alkylcarbonate, die dem eingesetzten Alkohol entsprechen, zu verstehen.

Nach dieser destillativen Aufarbeitung liegen die erfindungsgemäß Diurethane in einer ca. 99 %iger Reinheit vor. Als einziges nennenswertes Nebenprodukt ist lediglich noch das dem eingesetzten Alkohol ensprechende Harnstoffderivat der Formel

ROCONH-R'-NHCONH-R'-NHCOOR

zu nennen, das durch präparative Gelpermeationschromatgraphie isoliert und NMR-spektroskopisch identifiziert werden kann. Gewünschtenfalls kann das Verfahrensprodukt auch durch eine Hochvakuumdestillation von den geringen Mengen dieses Nebenprodukts befreit werden. Dies ist jedoch im allgemeinen wegen der geringen Konzentration dieser "Verunreinigung" nicht erforderlich.

Das nach der destillativen Entfernung der Leicht- und Mittelsieder als Destillationsrückstand in ca. 99 %iger Reinheit anfallende Diurethan kann der an sich bekannten thermischen Urethanspaltung zugeführt werden. Zusätzliche aufwendige und kostenintensive Reinigungsschritte wie z.B. eine vollständig Destillation des Diurethans sind nicht erforderlich. Das in Spuren (bis zu 1 %) vorliegende Harnstoffderivat der zuletztgenannten allgemeinen Formeln kann nach der Ausschleusung aus der thermischen Urethanspaltung unter Wiederverwendung beim erfindungsgemäßen Verfahren recyclisiert werden.

Das erfindungsgemäße Verfahren kann selbstverständlich auch kontinuierlich, beispielsweise unter Verwendung geeigneter Rührkasselkassaden durchgeführt werden.

Allen Varianten des erfindungsgemäßen Verfahrens gemeinsam ist, daß vorzugsweise auf die Verwendung von Katalysatoren ebenso wie auf die Verwendung von Inertgas zwecks Austreibens des Ammoniaks verzichtet wird.

In dem nachfolgenden Beispiel beziehen sich alle Prozentangaben auf das Gewicht.

## Beispiel

Reaktor: 2 l-Rührautoklav mit Mantelheizung und aufgesetzter Füllkörperkolonne (Länge: 50 cm, Durchmesser: 3 cm, Füllkörper: 4x4 mm VA-Stahl Maschendrahtringe), Schlangenkühler als Kondensator, Druckhalteventil am Kopf des Kühlers, Kühler und Druckhalteventil beheiz- und thermostatisierbar.

In den Druckbehälter werden 191 g (3,18 Mol) Harnstoff in 452 g (6,1 Mol) n-Butanol vorgelegt und bei einer auf 70°C eingestellten Kühlertemperatur unter Rühren auf 220°C erhitzt. Das Druckhalteventil wird so eingestellt, daß Ammoniak entweichen kann, während Butanol am Rückfluß siedet. Bis zur Gleichgewichtseinstellung nach ca. 1 Stunde werden 55 % der theoretisch zu erwartenden Ammoniakmenge entnommen. Dann wird eine Lösung von 168 g (1,45 Mol) 1,6-Diaminohexan (HDA) in 200 g (2,7 Mol) n-Butanol innerhalb 2 Stunden zudosiert und anschließend bei 220°C nachgerührt. Die Ammoniakabspaltung läßt nach 1 Stunde nach. Es wird noch weitere 8 Stunden nachgerührt, bis praktisch kein Ammoniak mehr entweicht. Anschließend wird das Reaktionsgemisch der destillativen Aufarbeitung zugeführt.

Zur destillativen Aufarbeitung des Reaktionsgemischs wird zunächst n-Butanol im Vakuum (15 mbar, Ölbadtemperatur 90°C) weitgehend abdestilliert. Es verbleiben 462 g Rückstand, der 95,0 % Hexamethylendibutylurethan enthält, was einer Ausbeute, bezogen auf eingesetztes Diaminohexan von 95,9 %, entspricht (Supercritical Fluid Chromatography, interner Standard). Gaschromatographisch (interner Standard) lassen sich als Nebenkomponenten 2,3 % Carbamidsäurebutylester, 1,3 % Dibutylcarbonat, 0,1 % N-Butylcarbamidsäurebutylester und 1,1 % Butanol bestimmen. Die Summe dieser Komponenten ergibt 99,8 %. Oligo-und Polyharnstoffe sind nicht nachweisbar.

Die genannten, flüchtigen Komponenten (Mittelsieder) können im Vakuum bei 140°C/0,05 mbar vollständig entfernt werden. Das so erhaltene Hexamethylendibutylurethan weist eine Reinheit von 99 % auf. Das Diurethan ist in dieser Form nach bekannten Verfahren, wie sie z.B. in EP-A-00 61 013, EP-A-00 92 738 oder EP-A-05 42 106 beschrieben sind, problemlos spaltbar.

Als Nebenprodukt ist lediglich ein Harnstoffderivat folgender Struktur erhalten:
$$BuOCONH-(CH_2)_6-NHCONH-(CH_2)_6-NHCOOBu.$$

Es kann durch präparative Gelpermeationschromatographie isoliert und NMR-spektroskopisch identifiziert werden. Oligo- und Polyharnstoffe sind nicht nachweisbar.

Durch eine Hochvakuumdestillation kann das Nebenprodukt (Harnstoffderivat) abgetrennt werden und unter Zusatz von Harnstoff und Butanol unter Einhaltung der oben angegebenen Stöchiometrie für HDA: Harnstoff:Butanol und der oben gewählten Reaktionsbedingungen recyclisiert werden.

## Patentansprüche

1. Verfahren zur Herstellung von Diurethanen der Formel (I)

$$R-O-CO-NH-R'-NH-CO-O-R \qquad (I)$$

unter Verwendung von Diaminen der Formel (II)

$$H_2N-R'-NH_2 \qquad (II)$$

Alkoholen der Formel (III)

$$R-OH \qquad (III)$$

und Harnstoff als Ausgangsmaterialien, wobei

R  für den Rest steht, wie er durch Entfernung der Hydroxylgruppe aus einem einwertigen Alkohol mit einem Siedepunkt unter Normaldruck von unter 180°C erhalten wird und

R'  für einen zweiwertigen (cyclo)aliphatischen Kohlenwasserstoffrest mit 2 bis 18 Kohlenstoffatomen steht, mit der Maßgabe, daß zwischen den beiden Stickstoffatomen mindestens 2 Kohlenstoffatome angeordnet sind,

dadurch gekennzeichnet, daß man

in einer ersten Stufe Harnstoff mit Alkohol der Formel (III) unter Einhaltung eines Molverhältnisses von Alkohol zu Harnstoff von mindestens 1:1, bei 150 bis 300°C unter Abspaltung und Entfernung aus dem Reaktionsgemisch von mindestens 10 Mol-% Ammoniak bezogen auf die molare Menge des eingesetzten Harnstoffs umsetzt, anschließend
in einer zweiten Stufe in diese Reaktionsmischung das Diamin der Formel (II), gegebenenfalls in in Alkohol der Formel (III) gelöster Form eindosiert und unter kontinuierlicher Freisetzung von Ammoniak bei 150 bis 300°C umsetzt und
schließlich in einer dritten Stufe das Reaktionsgemisch destillativ von flüchtigen Bestandteilen befreit.

2. Verfahren gemäß Anspruch 1, dadurch gekennzeichnet, daß man als Diamin der Formel (II) Hexandiamin-1,6, 3-Aminomethyl-3,5,5-trimethylcyclohexylamin oder Bis-(4-aminocyclohexyl)-methan verwendet.

3. Verfahren gemäß Anspruch 1 und 2, dadurch gekennzeichnet, daß man als Alkohol der Formel (III) n-Butanol verwendet.

4. Verwendung der gemäß Anspruch 1 bis 3 erhältlichen Diurethane als Ausgangsmaterial zur Herstellung der entsprechenden Diisocyanate durch thermische Urethanspaltung.

## Claims

1. Process for the preparation of diurethanes of the formula (I)

$$R-O-CO-NH-R'-NH-CO-O-R \qquad (I)$$

with the use as starting materials of diamines of the formula (II)

$$H_2N-R'-NH_2 \qquad (II),$$

alcohols of the formula (III)

$$R-OH \qquad (III)$$

and urea, wherein

R  stands for the radical obtained by removing the hydroxyl group from a monohydric alcohol having a boiling point of less than 180°C at standard pressure, and

R'  stands for a bivalent (cyclo)aliphatic hydrocarbon radical having 2 to 18 carbon atoms, with the proviso that there are arranged between the two nitrogen atoms at least 2 carbon atoms,

characterised in that

in a first stage urea is reacted with alcohol of the formula (III), observing a molar ratio of alcohol to urea of at least 1 : 1, at from 150 to 300°C with cleavage and removal from the reaction mixture of at least 10 mol.% ammonia, calculated on the molar quantity of urea used,

in a second stage the diamine of the formula (II), optionally in the form of a solution in alcohol of the formula (III), is dispensed into the latter reaction mixture and is reacted at from 150 to 300°C with continuous release of ammonia, and

finally, in a third stage, volatile constituents are removed from the reaction mixture by distillation.

2. Process according to Claim 1, characterised in that 1,6-hexanediamine, 3-aminomethyl-3,5,5-trimethylcyclohexylamine or bis(4-aminocyclohexyl) methane is used as the diamine of the formula (II).

3. Process according to Claims 1 and 2, characterised in that n-butanol is used as the alcohol of the formula (III).

4. Use of the diurethanes obtainable according to Claims 1 to 3 as a starting material for preparing the corresponding diisocyanates by thermal cleavage of urethane.

## Revendications

1. Procédé de préparation de diuréthanes de formule (I)

$$R-O-CO-NH-R'-NH-CO-O-R \qquad (I)$$

au moyen de diamines de formule (II)

$$H_2N-R'-NH_2 \qquad (II)$$

d'alcools de formule (III)

$$R-OH \qquad (III)$$

et d'urée comme matières premières, où

R représente le reste qui est obtenu par élimination du groupe hydroxyle d'un monoalcool ayant un point d'ébullition inférieur à 180°C sous la pression normale et

R' représente un reste hydrocarboné (cyclo)aliphatique divalent ayant 2 à 18 atomes de carbone, à condition qu'au moins deux atomes de carbone soient disposés entre les deux atomes d'azote,

caractérisé en ce que

dans une première étape, on fait réagir de l'urée avec un alcool de formule (III) en maintenant un rapport molaire de l'alcool à l'urée d'au moins 1:1, entre 150 et 300°C, avec clivage et élimination d'au moins 10 mol% d'ammoniac du mélange réactionnel, par rapport à la quantité molaire de l'urée utilisée,

puis, dans une deuxième étape, on introduit dans ce mélange réactionnel la diamine de formule (II), éventuellement sous forme dissoute dans un alcool de formule (III), et on la fait réagir avec libération continue d'ammoniac entre 150 et 300°C, et

enfin, dans une troisième étape, on débarrasse le mélange réactionnel des constituants volatils par distillation.

2. Procédé selon la revendication 1, caractérisé en ce que l'on utilise comme diamine de formule (II) l'hexane-1,6-diamine, la 3-aminométhyl-3,5,5-triméthylcyclohexylamine ou le bis-(4-aminocyclohexyl)-méthane.

3. Procédé selon les revendications 1 et 2, caractérisé en ce que l'on utilise le n-butanol comme alcool de formule (III).

4. Utilisation des diuréthanes qui peuvent être obtenus selon les revendications 1 à 3 comme matière première pour la préparation des diisocyanates correspondants par clivage thermique d'uréthane.